# EUROPEAN PATENT APPLICATION

(11) **EP 4 585 683 A1**
(43) Date of publication of application: **16.07.2025**
(21) Application number: 23861915.9
(22) Date of filing: 23.05.2023
(51) Int. Cl.: C12N 15/11, C07H 21/00, A61K 39/29, A61K 39/39, A61P 31/12

(54) **PHARMACEUTICAL COMPOSITION FOR PREVENTING HEPATITIS B VIRUS INFECTION AND USE THEREOF**

(30) Priority: 07.09.2022 CN 202211087949
(71) Applicant: Grand Theravac Life Science (Nanjing) Co., Ltd., Nanjing, Jiangsu 210032 (CN)
(72) Inventor: LI, Jianqiang, Nanjing, Jiangsu 210032 (CN); GE, Jun, Nanjing, Jiangsu 210032 (CN); SUN, Jiaojiao, Nanjing, Jiangsu 210032 (CN); ZHOU, Tong, Nanjing, Jiangsu 210032 (CN); GU, Yue, Nanjing, Jiangsu 210032 (CN); HUANG, Hongying, Nanjing, Jiangsu 210032 (CN); CHEN, Xiaoxiao, Nanjing, Jiangsu 210032 (CN); CHEN, Yue, Nanjing, Jiangsu 210032 (CN); HE, Yandong, Nanjing, Jiangsu 210032 (CN); WANG, Cheng, Nanjing, Jiangsu 210032 (CN)
(74) Representative: Harrison IP Limited
(86) International application number: PCT/CN2023/095721
(87) International publication number: WO 2024/051211

(57) **Abstract**

The present invention provides a pharmaceutical composition for preventing hepatitis B virus infection and use thereof. The pharmaceutical composition comprises i) a hepatitis B virus surface antigen, a fragment thereof with antigenic activity and/or a variant thereof with antigenic activity; and ii) an immunostimulatory composition, which comprises an aluminum adjuvant and a CpG oligodeoxynucleotide, wherein the sequence of the CpG oligodeoxynucleotide comprises two or more copies of 5'-TTCGTT-3' motif or 5'-TCGTCGTCG-3' motif. The experimental results show that the components contained in the immunostimulatory composition can have a synergistic effect, thereby greatly enhancing the immune effect on hepatitis B virus surface antigen; only two immunizations can produce a good immune effect, and produce the antibody earlier than the existing vaccines and maintain a high level of antibody titer for a longer time.

## Description

### Technical Field

The present invention relates to the field of biopharmaceuticals, and in particular to a pharmaceutical composition for preventing hepatitis B virus infection and use thereof.

### Background Art

Hepatitis B virus (HBV) infection is one of the serious public health problems worldwide. HBV infection is an important cause of chronic hepatitis B, liver cirrhosis and hepatocellular carcinoma (Fattovich G. J. Hepatol. 2008; 48:335-352). Hepatitis B protein vaccines, which are widely used today, reach the purpose of prophylaxis by inducing humoral immunity and producing protective neutralizing antibodies.

At present, the prophylactic hepatitis B vaccines on the market use aluminum hydroxide (Al(OH)₃) as an adjuvant, which is able to enhance Th2 immune response and humoral immunity, and induce the body to produce and secrete protective antibodies. However, aluminum hydroxide mainly stimulates the production of Th2-related antibodies (including IgE), but cannot induce the production of Th1 cell immune response, stimulate Th1 activity, enhance cellular immunity (CTL), block the activation of CD8+CTL.

Studies have found that CpG oligodeoxynucleotide (CpG-ODN) adjuvants have an immune-enhancing effect, can bind to TLR9 (Toll-like receptor 9) in cells, induce an immune response dependent on Th1 pathway, activate B cells to differentiate into plasma cells that secrete antigen-specific antibodies, and stimulate the expression of IFN-y, thereby promoting cellular immune response and increasing the immune effects in non-immune, low-immune response and immunosuppressed populations. Therefore, the combined use of a CpG oligodeoxynucleotide and an aluminum hydroxide adjuvant can provide better immune effects, such as the combination of CpG and aluminum hydroxide mentioned in the patent application CN105214083A.

However, studies have also found that CpG adjuvants have structural diversity, and different types of CpG adjuvants have great differences in the effects in practical applications, and high uncertainty of druggability results. How to screen out adjuvant compositions with better immune-enhancing effects and to improve the prophylactic efficacies of hepatitis B vaccines are still urgent issues that need to be resolved in this field.

### Contents of the Invention

In order to overcome the deficiencies in the prior art, it is an object of the present invention to provide a pharmaceutical composition for preventing hepatitis B virus infection and use thereof, which has a shorter onset time and a better immunological effect in preventing hepatitis B virus infection.

The technical solutions of the present invention for achieving the above object are as follows:
A pharmaceutical composition for preventing hepatitis B virus infection comprising:
i) a hepatitis B virus surface antigen, a fragment thereof with antigenic activity and/or a variant thereof with antigenic activity; and
ii) an immunostimulatory composition, which comprises an aluminum adjuvant and a CpG oligodeoxynucleotide, wherein the sequence of the CpG oligodeoxynucleotide comprises two or more copies of 5'-TTCGTT-3' motif or 5'-TCGTCGTCG-3' motif.

In some embodiments of the present invention, the CpG oligodeoxynucleotide may be one or more selected from a group consisting of the CpG oligodeoxynucleotides as shown in SEQ ID NOs: 2 to 4.

Preferably, the CpG oligodeoxynucleotide is a CpG oligodeoxynucleotide as shown in SEQ ID NO: 2.

In some embodiments of the present invention, the aluminum adjuvant may be one or more selected from a group consisting of aluminum hydroxide, aluminum phosphate, aluminum sulfate, aluminum ammonium sulfate or aluminum potassium sulfate, preferably aluminum hydroxide.

In some embodiments of the present invention, the CpG oligodeoxynucleotide may contain a phosphorothioate linkage.

Preferably, the CpG oligodeoxynucleotide is a perthio-oligodeoxynucleotide.

In some embodiments of the present invention, the weight ratio of the CpG oligodeoxynucleotide to the aluminum adjuvant is 10-200: 37.5, preferably 60-200: 37.5, further preferably 200: 37.5.

According to the present invention, the weight ratio of the CpG oligodeoxynucleotide to the aluminum adjuvant is selected from 10: 37.5, 20: 37.5, 30: 37.5, 40: 37.5, 50: 37.5, 60: 37.5, 70: 37.5, 80: 37.5, 90: 37.5, 100: 37.5, 110: 37.5, 120: 37.5, 130: 37.5, 140: 37.5, 150: 37.5, 160: 37.5, 170: 37.5, 180: 37.5, 190: 37.5 or 200: 37.5.

In some embodiments of the present invention, the hepatitis B virus surface antigen has an amino acid sequence as shown in SEQ ID NO: 1.

In some embodiments of the present invention, the weight ratio of Component i) to Component ii) in the pharmaceutical composition is 1: 47.5-237.5, preferably 1: 97.5-237.5, further preferably 1: 237.5.

In some embodiments of the present invention, the pharmaceutical composition is a unit formulation, wherein the amount of Component i) is ≥5 µg/dose, and Component ii) has an amount of Al(OH)₃ ≥187.5 µg/dose and an amount of CpG ≥300 µg/dose.

Preferably, the amount of CpG is 300-1000 µg/dose.

Preferably, the amount of CpG is 1000 µg/dose.

According to the present invention, the amount of CpG may be 300µg/dose, 350µg/dose, 400µg/dose, 450µg/dose, 500µg/dose, 550µg/dose, 600µg/dose, 650µg/dose, 700µg/dose, 750µg/dose, 800µg/dose, 850µg/dose, 900µg/dose, 950µg/dose or 1000µg/dose.

In some embodiments of the present invention, the pharmaceutical composition may further comprise: iii) a pharmaceutically acceptable carrier and/or excipient.

In some embodiments of the present invention, the pharmaceutically acceptable carrier and/or excipient may be one or more selected from a group consisting of water, buffered aqueous solution, isotonic saline solution, wetting agent or emulsifier. Preferably, the pharmaceutically acceptable carrier and/or excipient is one or more selected from a group consisting of phosphate buffered saline (PBS), glucose, mannitol, dextrose, lactose, starch, magnesium stearate, cellulose, magnesium carbonate, 0.3% v/v glycerol aqueous solution, hyaluronic acid, ethanol, polyalkylene glycol (e.g., polypropylene glycol) or triglyceride.

The type of carrier used in the pharmaceutical composition depends in particular on whether the pharmaceutical composition according to the present invention is formulated for oral, nasal, intradermal, subcutaneous, intramuscular or intravenous administration. The pharmaceutical composition, vaccine or pharmaceutical preparation according to the present invention can be administered by any suitable route, for example, by oral, nasal, intradermal, subcutaneous, intramuscular or intravenous route.

In some embodiments of the present invention, the dosage form of the pharmaceutical composition may be a lyophilized powder injection or an injectable solution.

Another object of the present invention is to provide a hepatitis B prophylactic vaccine comprising the pharmaceutical composition as described above.

Still another object of the present invention is to provide use of the pharmaceutical composition in the manufacture of a medicament, such as a vaccine, for preventing hepatitis B virus infection and/or hepatitis B virus-mediated disease.

In some embodiments of the present invention, the hepatitis B virus infection and/or hepatitis B virus-mediated disease is one or more selected from a group consisting of hepatitis B, liver cirrhosis or liver cancer.

The experimental results of the present invention show that the pharmaceutical composition provided can induce the generation of a significant humoral immune response, and produce an immune response effect earlier and faster than a single adjuvant system: a higher level of HBsAb positive conversion rate can be induced earlier after a primary immunization (wk02), and a higher level of HBsAb antibody can be produced after a secondary immunization.

The CpG adjuvant and the aluminum adjuvant in the pharmaceutical composition of the present invention have a good synergistic effect, and the combined use of the two adjuvants can improve the protective effect of the hepatitis B prophylactic vaccine.

In addition, in the cynomolgus monkey experiment, compared with the three-shot immunization scheme of the commercially available hepatitis B vaccine, the two-shot immunization scheme of the present invention can achieve the same effect and has a shorter immune onset time, indicating that in the practical application process, the pharmaceutical composition of the present invention can play a preventive and protective role in human body in a shorter period of time.

### Brief Description of the Drawings

The embodiments of the present invention are described in detail with reference to the accompanying drawings, wherein:
Fig. 1 shows the levels of the HBsAb antibody in mouse sera;
Fig. 2 shows the levels of the HBsAb antibody in cynomolgus monkey sera.

### Modes for Carrying Out the Invention

The present invention is further described in detail below in combination with the specific embodiments, wherein the given examples are for illustrative purposes only, and not intended to limit the scope of the invention.

### Example 1 Preparation of pharmaceutical composition

### 1. Expression of HBsAg protein

The HBsAg protein has an amino acid sequence as shown in SEQ ID NO: 1. It was prepared from HBsAg gene recombinant yeast cells, which can be selected from Hansenula yeast, Saccharomyces cerevisiae yeast and Pichia pastoris yeast, preferably Hansenula yeast. The disclosure of Chinese patent application CN108330145A was referred to for the specific preparation procedure, wherein the HBsAg gene recombinant Hansenula yeast cells were fermented and cultured for harvesting the cells. The purification was carried out by cell disruption treatment, silica gel adsorption, column chromatography, TFF, etc. The purified HBsAg protein was used for the preparation of a pharmaceutical composition.

### 2. Synthesis of CpG oligodeoxynucleotides

CpG oligodeoxynucleotides were synthetically prepared oligodeoxynucleotide sequence fragments, which have two or more copies of 5'-TTCGTT-3' motif or 5'-TCGTCGTCG-3' motif in the sequence. The CpG oligodeoxynucleotide of the present invention is shown in any one of SEQ ID NO: 2, SEQ ID NO: 3 or SEQ ID NO: 4, which was prepared with reference to Chinese patent CN104043120B. The following examples illustrate the effect of the immunostimulatory composition of the present invention taking the CpG oligodeoxynucleotide as shown in SEQ ID NO: 2 as an example.

### Example 2 Detection method of positive conversion rate of Hepatitis B virus surface antibody (HBsAb)

### 1. Experimental procedure:

40 female BALB/c mice were used and divided into 5 groups, Groups A-E (8 mice/group); Group A was administered HBsAg 1µg/mouse; Group B was administered HBsAg 1µg/mouse, and Al(OH)₃ 37.5µg/mouse; Group C was administered HBsAg 1µg/mouse, and CpG 10µg/mouse; Group D was administered HBsAg 1µg/mouse, Al(OH)₃ 37.5µg/mouse, and CpG 10µg/mouse; and Group E was administered a control vaccine (Dalian Hanxin), HBsAg 1µg/mouse, and Al(OH)₃ 37.5µg/mouse. Immunization was performed by intramuscular injection twice at wk (week) 00 and wk04, respectively. Blood was collected at wk02, wk04, wk06, wk08, wk12, wk16 and wk20 after the administration, respectively. The sera of wk02 and wk04 were tested for HBsAb positive conversion rate, and the sera of wk04, wk06, wk08, wk12, wk16 and wk20 were tested for HBsAb antibody level, respectively.

The HBsAb positive conversion rates of the hepatitis B virus surface antibody in the mouse serum samples were qualitatively detected using a detection kit for hepatitis B virus surface antibody (enzyme-linked immunosorbent assay, Shanghai Kehua Bio-engineering Co., Ltd.).

### 2. Experimental results:

The results of the HBsAb positive conversion rate in the mouse serum of each group at different detection times are shown in Table 1. The results show that at two weeks after the first vaccination (wk02), the HBsAb positive conversion rate of Group D containing dual adjuvant components was the highest, with an antibody positive conversion rate of 50%, while the antibody positive conversion rates of the other groups were relatively low, and the antibody positive conversion rate of Group E, the control vaccine group, was only 12.5%; at four weeks after the first vaccination (wk04), the HBsAb positive conversion rate in the serum of each group increased, with the antibody positive conversion rates of Groups B, D and E all reaching 100%.

The experimental results show that the HBsAb positive conversion rate (%) of Group D at wk02 was much higher than those of Groups B and C; that is, in the present invention, the CpG adjuvant and the aluminum adjuvant had a good synergistic effect, and the combined use of the two adjuvants could improve the protective effect of the hepatitis B preventive vaccine earlier.

**Table 1 HBsAb positive conversion rates in mouse serum**

| Group | Number (mouse) | Vaccine components | HBsAb positive conversion rate (%) at wk02 | HBsAb positive conversion rate (%) at wk04 |
|---|---|---|---|---|
| A | 8 | HBsAg | 0 (2/8) | 50 (4/8) |
| B | 8 | HBsAg+Al(OH)₃ | 25 (2/8) | 100 (8/8) |
| C | 8 | HBsAg+CpG | 0 (0/8) | 62.5 (5/8) |
| D | 8 | HBsAg+Al(OH)₃+CpG | 50 (4/8) | 100 (8/8) |
| E | 8 | Control vaccine | 12.5 (1/8) | 100 (8/8) |

### Example 3 Detection of level of HBsAb antibody in mouse serum

### 1. Experimental procedure:

Both the animal immunization and serum collection were carried out with reference to Example 2. The mouse serum samples were diluted appropriately with sterile PBS (dilution times n), and the HBsAb levels in the mouse serum samples were quantitatively determined using the ARCHITECT hepatitis B virus surface antibody assay kit through chemiluminescent microparticle immunoassay technology (CMIA).

### 2. Experimental results:

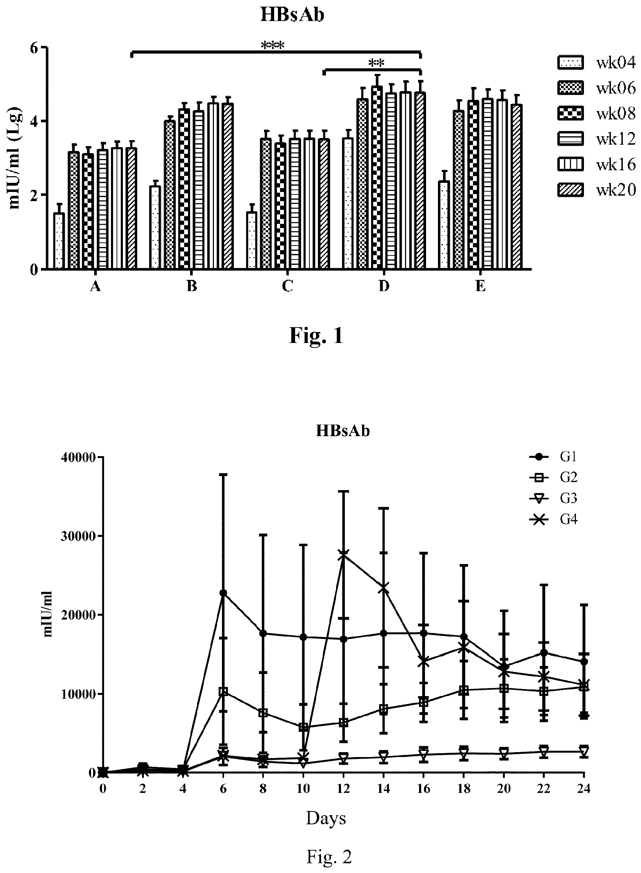

As shown in Fig. 1, the HBsAb antibody level in the serum of each group gradually increased over time, and at wk16, the HBsAb antibody reached a relatively high level and tended to be stable. The trend of the induced HBsAb antibody levels among Groups A-D was: Group D>Group B>Group C>Group A, and at wk16, the HBsAb antibody levels of Groups A-E were 3.27, 4.48, 3.52, 4.78 and 4.57 Lg, respectively, showing the statistical differences: as shown in Fig. 1, *** represents the data at week 20 of Group D versus that of Group A, p < 0.001, ** represents the data at week 20 of group D versus that of Group C, p < 0.01. The antibody level of Group D was the highest, and the HBsAb antibody level of group E, the control vaccine group, was always lower than that of Group D, indicating that the immune response level and sustained protection effect of the pharmaceutical composition provided by the present invention were superior to those of the commercially available hepatitis B vaccine.

### Example 4 Detection of level of HBsAb antibody in cynomolgus monkey serum

### 1. Experimental procedure:

Cynomolgus monkeys (Guangdong Blooming Spring Biotechnology Development Co., Ltd.), 12 males and 12 females, aged 2-5 years, were divided into 4 groups, G1-G4 (6 monkeys per group, half male and half female).

**Table 2 Experimental animal grouping**

| Group | Vaccine components | Components µg/monkey | | |
|---|---|---|---|---|
| | | HBsAg | Al(OH)₃ | CpG |
| G1 | HBsAg+Al(OH)₃+CpG | 5 | 187.5 | 1000 |
| G2 | HBsAg+Al(OH)₃+CpG | 5 | 187.5 | 300 |
| G3 | HBsAg+Al(OH)₃+CpG | 5 | 187.5 | 100 |
| G4 | Control vaccine (GSK) | 5 | 361 | |

Groups G1-G3 were immunized by intramuscular injection at wk00 and wk04, respectively, for a total of 2 immunizations; Group G4 was immunized by intramuscular injection at wk00, wk04 and wk10, respectively, for a total of 3 immunizations; the intramuscular injection was used through a single site administration to the quadriceps muscle group or the gluteal muscle group of the hind limb. Blood was collected every two weeks after the administration until wk24, and the sera collected at wk02-wk24 were tested for the HBsAb antibody level using the same detection method as in Example 2.

### 2. Experimental results:

As shown in Fig. 2, Groups G1-G3 were immunized with the pharmaceutical compositions containing different doses of CpG at wk00 and wk04, respectively. The induced levels of HBsAb antibody all reached higher levels at wk06, with average antibody levels of 22763, 10275 and 2055 mIU/ml, respectively. Thereafter, the antibody levels showed a certain extent of change, and tended to stabilize at wk18-wk24, with average antibody levels of 14047, 10871 and 2641 mIU/ml at wk24 respectively. During the entire experimental period, the trend of induced HBsAb antibody in Groups G1-G3 was: G1>G2>G3, that is, there was a positive correlation between the antibody level and the CpG dose.

Group G4 was immunized with the control vaccine at wk00, wk04 and wk10, respectively, and the level of the induced HBsAb antibody reached a peak at wk12, with an average antibody level of 27580 mIU/ml, and then showed a certain decreasing trend; and the antibody level tended to stabilize at wk18-wk24, with an average antibody level of 11138 mIU/ml at wk24.

During the entire experimental period, comparison between the HBsAb antibody levels induced by Groups G1-G3 immunized with the double-adjuvant vaccine and that induced by Group G4 immunized with the control vaccine indicates, during the period of wk06-wk10 after the second immunization, the immunized groups showed a trend of G1>G2>G4>G3; at wk10, after the third immunization, the antibody level of Group G4 increased significantly; during the period of wk12-wk14, the immunized groups showed a trend of G4>G1>G2>G3; thereafter, the antibody level of Group G4 showed a certain decreasing trend; and during the period of wk16-wk24, the immunized groups showed a trend of G1>G4>G2>G3, wherein the antibody levels of Groups G2 and G4 were equivalent at wk24 without significant difference.

Compared with the commercially available hepatitis B vaccine, during the two immunizations, the antibody level induced by the double-adjuvant vaccine containing a higher CpG dose (Group G1) was higher than that of the control vaccine (Group G4); after the third booster immunization of the control vaccine, the antibody level induced by it increased significantly, but after the antibody level achieved a relative stability, its antibody level was still lower than that of the double-adjuvant vaccine in Group G1.

In summary, the pharmaceutical composition provided by the present invention has a significant immune effect, and has its experimental effect on mice being superior to the prior art, and its experimental effect on cynomolgus monkeys being superior to the existing three-shot commercial vaccine. The hepatitis B vaccine prepared from the pharmaceutical composition provided by the present invention has significant advantages.

The above-mentioned examples only represent several embodiments of the present invention by relatively specific and detailed description, but cannot be construed as a limitation of the scope of the present invention. It should be noted that for those of ordinary skill in the art, without departing from the concept of the present invention, several modifications and improvements can be made, which all fall within the scope of the present invention. Therefore, the scope of the present invention shall be subject to the attached claims.

## Claims

1. A pharmaceutical composition for preventing hepatitis B virus infection comprising:
i) a hepatitis B virus surface antigen, a fragment thereof with antigenic activity and/or a variant thereof with antigenic activity; and
ii) an immunostimulatory composition, which comprises an aluminum adjuvant and a CpG oligodeoxynucleotide, wherein the sequence of the CpG oligodeoxynucleotide comprises two or more copies of 5'-TTCGTT-3' motif or 5'-TCGTCGTCG-3' motif.

2. The pharmaceutical composition according to claim 1, wherein the CpG oligodeoxynucleotide is one or more selected from a group consisting of the CpG oligodeoxynucleotides as shown in SEQ ID NOs: 2 to 4;
preferably, the CpG oligodeoxynucleotide is a CpG oligodeoxynucleotide as shown in SEQ ID NO: 2.

3. The pharmaceutical composition according to claim 1 or 2, wherein the aluminum adjuvant is one or more selected from a group consisting of aluminum hydroxide, aluminum phosphate, aluminum sulfate, aluminum ammonium sulfate or aluminum potassium sulfate, preferably aluminum hydroxide.

4. The pharmaceutical composition according to any one of claims 1 to 3, wherein the CpG oligodeoxynucleotide comprises a phosphorothioate inter-nucleoside linkage;
preferably, the CpG oligodeoxynucleotide is a perthio-oligodeoxynucleotide.

5. The pharmaceutical composition according to any one of claims 1 to 4, wherein the weight ratio of the CpG oligodeoxynucleotide to the aluminum adjuvant is 10-200: 37.5, preferably 60-200: 37.5, further preferably 200: 37.5.

6. The pharmaceutical composition according to any one of claims 1 to 5, wherein the hepatitis B virus surface antigen has an amino acid sequence as shown in SEQ ID NO: 1.

7. The pharmaceutical composition according to any one of claims 1 to 6, wherein the weight ratio of Component i) to Component ii) in the pharmaceutical composition is 1: 47.5-237.5, preferably 1: 97.5-237.5, further preferably 1: 237.5.

8. The pharmaceutical composition according to any one of claims 1 to 7, wherein the pharmaceutical composition is a unit formulation, wherein the amount of Component i) is ≥5 µg/dose, and Component ii) has an amount of Al(OH)₃ ≥187.5 µg/dose and an amount of CpG ≥300 µg/dose;
preferably, the amount of CpG is 300-1000 µg/dose; and
preferably, the amount of CpG is 1000 µg/dose.

9. The pharmaceutical composition according to any one of claims 1 to 8, wherein the pharmaceutical composition further comprises: iii) a pharmaceutically acceptable carrier and/or excipient;
preferably, the pharmaceutically acceptable carrier and/or excipient is one or more selected from a group consisting of water, buffered solution, isotonic saline solution, wetting agent or emulsifier;
further preferably, the pharmaceutically acceptable carrier and/or excipient is one or more selected from a group consisting of phosphate buffered saline, glucose, mannitol, dextrose, lactose, starch, magnesium stearate, cellulose, magnesium carbonate, 0.3% v/v glycerol aqueous solution, hyaluronic acid, ethanol, polyalkylene glycol (e.g., polypropylene glycol) or triglyceride.

10. The pharmaceutical composition according to any one of claims 1 to 9, wherein the dosage form of the pharmaceutical composition is a lyophilized powder injection or an injectable solution.

11. A hepatitis B prophylactic vaccine comprising the pharmaceutical composition according to any one of claims 1 to 10.

12. Use of the pharmaceutical composition according to any one of claims 1 to 10 in the manufacture of a medicament, such as a vaccine, for preventing hepatitis B virus infection and/or hepatitis B virus-mediated disease.

13. Use according to claim 12, wherein the hepatitis B virus infection and/or hepatitis B virus-mediated disease is one or more selected from a group consisting of hepatitis B, liver cirrhosis or liver cancer.
